# EUROPEAN PATENT SPECIFICATION

(11) **EP 3 964 127 B1**
(45) Date of publication and mention of the grant of the patent: **01.10.2025**
(21) Application number: 20830829.6
(22) Date of filing: 20.06.2020
(51) Int. Cl.: A61B 5/00, A61B 5/024, A61B 5/1455

(54) **WEARABLE DEVICE AND PHOTOELECTRIC PULSE SENSOR COMPONENT**
WEARABLE-VORRICHTUNG UND SENSORKOMPONENTE MIT PHOTOELEKTRISCHEM IMPULS
DISPOSITIF POUVANT ÊTRE PORTÉ ET COMPOSANT DE CAPTEUR D'IMPULSION PHOTOÉLECTRIQUE

(30) Priority: 24.06.2019 CN 201910550081
(43) Date of publication of application: 09.03.2022
(73) Proprietor: Huawei Technologies Co., Ltd., Longgang District Shenzhen, Guangdong 518129 (CN)
(72) Inventor: XI, Yi, Shenzhen, Guangdong 518129 (CN); SUN, Shiyou, Shenzhen, Guangdong 518129 (CN); SU, Dan, Shenzhen, Guangdong 518129 (CN)
(74) Representative: Maiwald GmbH
(86) International application number: PCT/CN2020/097282
(87) International publication number: WO 2020/259422

(56) References cited:
- EP-A1- 3 057 138
- WO-A1-2018/082211
- CN-A- 106 901 703
- CN-A- 107 095 644
- CN-A- 107 438 402
- CN-A- 109 152 530
- CN-A- 109 828 312
- CN-A- 110 393 514
- CN-U- 206 586 941
- US-A1- 2011 316 006
- US-A1- 2016 029 911
- US-A1- 2016 029 911
- US-A1- 2016 302 674

## Description

This application claims priority to Chinese Patent Application No. 201910550081.5, filed with the Chinese National Intellectual Property Administration on June 24, 2019 and entitled "WEARABLE DEVICE AND PHOTOELECTRIC PULSE SENSOR COMPONENT".

### TECHNICAL FIELD

The present invention relates to the field of electronic device technologies, and in particular, to a wearable device and a photoelectric pulse sensor component.

### BACKGROUND

A wearable device is a portable device that may be directly worn on a human body or integrated into clothes or an accessory of a person. With rapid development of electronic technologies, the wearable device may implement increasingly more functions such as sports monitoring, health monitoring, and sleep monitoring by detecting a parameter such as a heart rate and oxygen saturation of the human body.

In related technologies, the wearable device usually detects the parameter such as a heart rate and oxygen saturation by using a PPG (photoplethysmography, photoplethysmography) pulse wave method. The PPG is a method for measuring a parameter such as a heart rate and oxygen saturation based on photoelectric detection according to a principle that a blood volume changes with a pulse. For example, the wearable device may emit light to a wearing position on a human body, and then detect intensity of light reflected after the light is absorbed by blood and tissues of the human body, to record a blood volume change in a cardiac cycle, obtain a pulse waveform, and calculate the parameter such as a heart rate and oxygen saturation based on the obtained pulse waveform.

However, in a detection process of the wearable device, light loss is relatively severe. Consequently, less light enters the human body, and the intensity of the light reflected after the light is absorbed by the blood and the tissues of the human body is also correspondingly decreased. This affects a detection result of the parameter such as a heart rate and oxygen saturation, and reduces detection precision of the parameter such as a heart rate and oxygen saturation.

Document EP 3 057 138 A1 describes an optical sensor module, an optical sensing accessory, and an optical sensing device. An optical sensor module comprises a light source, a photodetector, and a substrate. The light source is configured to convert electric power into radiant energy and emit light to an object surface. Document CN 109 828 312 A describes an optical sensor which includes a substrate, light emitting units for emitting light with different wavelengths, and a photodetector. The substrate has at least one receiver for containing these light emitting units and a slot for containing the photodetector. A light guide structure of the optical sensor can be the receiver with a specific design, so that the light emitted by the light emitting units can be reflected towards a central axis of the photodetector.

Document US 2016/302674 A1 describes systems, methods and devices for reducing noise in health monitoring including monitoring systems. The methods and/or devices receivies a health signal and/or have at least one electrode or sensor for health monitoring.

Document US 2011/316006 A1 describes surface-textured encapsulations for use with light emitting diodes to provide for enhanced light output.

### SUMMARY

The present invention is defined in the independent claim. The dependent claims define the advantageous embodiments thereof.

The present invention provides a wearable device and a photoelectric pulse sensor component, to reduce a divergence angle of light emitted by a light emitting component in the wearable device, and improve light utilization, thereby improving detection precision of a heart rate and oxygen saturation, and reducing power consumption.

To achieve the foregoing objective, the following technical solutions are used in the present invention:
According to a first aspect, the present invention provides a wearable device. The wearable device includes a substrate; at least one light emitting component that is mounted on one side of the substrate and is configured to emit light on at least one optical wavelength band; and at least one lens disposed on an out-light side of the at least one light emitting component, where each lens corresponds to at least one light emitting component, and each lens is capable of reducing a divergence angle of light emitted by the at least one light emitting component corresponding to the lens. In this way, a light ray emitted by each light emitting component can be concentrated in the center, so that a large-angle light ray (namely, a light ray, where an included angle between the light ray and a normal line of an out-light surface of the light emitting component is relatively large) emitted by the light emitting component is effectively used, a depth at which the light ray emitted by the light emitting component enters a human body becomes deeper, and more light is absorbed by blood, that is, a proportion of desired light is increased. This improves detection precision of a heart rate and oxygen saturation, and reduces power consumption.

With reference to the first aspect, in a first possible design, the at least one lens is fastened to the substrate, a surface that is of each lens and that is connected to the substrate has at least one concave cavity, at least one accommodation space is surrounded by the at least one concave cavity and the substrate, and the at least one light emitting component corresponding to each lens is disposed in the at least one accommodation space. One or more light emitting components may be accommodated in each accommodation space. In this design, an overall thickness that exists in a direction perpendicular to the substrate after the lens and the substrate are assembled can be reduced. In addition, the light emitting component can be further protected by using the lens, so that the light emitting component is not vulnerable to external moisture, and a service life of the light emitting component is prolonged.

With reference to the first aspect, in a second possible design, the at least one lens is a Fresnel lens, and a protrusion and at least one circle of groove that has a sawtooth cross section and that is disposed around the protrusion are provided on a surface that is of the Fresnel lens and that faces away from at least one light emitting component corresponding to the Fresnel lens. In this design, a thickness of the lens in a direction perpendicular to the substrate can be reduced while a divergence angle of light emitted by the light emitting component is reduced, so that an internal structure of the wearable device is more compact, to miniaturize thae wearable device.

With reference to the first aspect, in a third possible design, a shape of the at least one lens is a part of an ellipsoid, an orthographic projection of the part of the ellipsoid on the substrate is an ellipse, and a major axis direction of the ellipse is parallel to a long side direction of a smallest rectangular area in which at least one light emitting component corresponding to the lens is located. Therefore, this helps reduce a size of the lens in a minor axis direction of the ellipse. In addition, a plurality of light emitting components can be sequentially arranged in the smallest rectangular area, and a divergence angle of light emitted by each light emitting component is effectively reduced by using the lens.

With reference to the first aspect, in a fourth possible design, each lens in the at least one lens includes at least two sub-lenses, each sub-lens is a part of a sphere or a part of an ellipsoid, an orthographic projection of the part of the sphere or the part of the ellipsoid on the substrate is a circle, each sub-lens corresponds to one light emitting component, and two adjacent sub-lenses partially overlap. This helps reduce a size of the lens in an arrangement direction of the at least two sub-lenses, to miniaturize the wearable device, and a divergence angle of light emitted by a corresponding light emitting component can be reduced by using each sub-lens. Therefore, this helps a light ray emitted by each light emitting component be concentrated in the center, so that a depth at which the light ray emitted by each light emitting component enters a human body becomes deeper, more light is absorbed by blood, and a proportion of desired light of each light emitting component is increased. This improves detection precision of a heart rate and oxygen saturation.

With reference to the first aspect, in a fifth possible design, a shape of the at least one lens is a part of a cylinder, the part of the cylinder is a part that is obtained through cutting off in a direction parallel to an axis direction of the cylinder, a cross section of the part of the cylinder in a direction perpendicular to the substrate and perpendicular to the axis direction of the cylinder is a part of a circle or a part of an ellipse, an orthographic projection of the part of the cylinder on the substrate is a rectangle, and a long side direction of the rectangle is parallel to a long side direction of a smallest rectangular area in which at least one light emitting component corresponding to the lens is located. In this way, a divergence angle, of light emitted by each light emitting component, in the direction perpendicular to the axis direction of the cylinder can be effectively reduced, and a size of the lens in the axis direction of the cylinder can be reduced.

With reference to the first aspect, in a sixth possible design, at least one lens is a convex lens, for example, a biconvex lens or a plano-convex lens. In this way, a divergence angle of light emitted by the light emitting component can be reduced by using a curved surface of the lens, so that a light ray emitted by each light emitting component is concentrated in the center. In addition, when the lens is a plano-convex lens, an overall thickness that exists in a direction perpendicular to the substrate after the lens and the substrate are assembled can be further reduced.

With reference to the first aspect, in a seventh possible design, a refractive index of each lens is greater than a refractive index of air. This helps increase an angle of refraction of a light ray emitted by the lens, and further reduce a divergence angle of light emitted by the light emitting component, so that more emergent light rays are concentrated in the center. Therefore, a large-angle light ray emitted by the light emitting component is effectively used, so that a depth at which the light ray emitted by the light emitting component enters a human body becomes deeper, and more light is absorbed by blood, that is, a proportion of desired light is increased, to further improve detection precision of a heart rate and oxygen saturation, and reduce power consumption.

With reference to the first aspect, in an eighth possible design, each lens is in contact with an out-light surface of the at least one light emitting component corresponding to the lens. In this way, more emergent light rays can be extracted from the light emitting component, so that a total reflection light ray of light generated by the light emitting component is not easily generated inside the light emitting component. This helps improve out-light efficiency of the light emitting component, reduce light loss, and improve light utilization.

With reference to the first aspect, in a ninth possible design, the at least one light emitting component includes at least one red light emitting diode, at least one green light emitting diode, and at least one infrared light emitting diode. The wearable device further includes: at least one photosensor mounted on the substrate, where the at least one photosensor is mounted on a same side of the substrate as the at least one light emitting component, and is configured to receive the light emitted by the at least one light emitting component, to generate a sensing signal; a control chip that is electrically connected to the at least one light emitting component and the at least one photosensor and is configured to: control a specified light emitting diode in the at least one light emitting component to emit light, receive and process the sensing signal generated by the at least one photosensor, and output the processed sensing signal; and a housing, where the substrate and the control chip are mounted in the housing, and a position that is on the housing and that corresponds to the at least one light emitting component and the at least one photosensor is transparent. In this design, components other than the housing of the wearable device are integrated into the housing. This has advantages of a simple structure and ease of portability. In addition, light emitted by the light emitting component may be incident into a target creature (for example, a human body) by using the transparent position on the housing, and light reflected by the human body is received by the photosensor, to detect a plurality of parameters of the target creature. For example, a resting heart rate, heart rate variability, sleep, and a blood pressure may be detected by using the red light emitting diode, the green light emitting diode, or the infrared light emitting diode; oxygen saturation may be detected by emitting light by the red light emitting diode and the infrared light emitting diode simultaneously; and an exercise heart rate may be detected by using the green light emitting diode.

With reference to the ninth possible design of the first aspect, in a tenth possible design, the at least one light emitting component includes two red light emitting diodes, two green light emitting diodes, and two infrared light emitting diodes. In a first direction, one red light emitting diode, one green light emitting diode, and one infrared light emitting diode are sequentially arranged in a row at intervals, and the other red light emitting diode, the other green light emitting diode, and the other infrared light emitting diode are sequentially arranged in another row at intervals. In a second direction, one red light emitting diode and one infrared light emitting diode are arranged in a column at intervals, two green light emitting diodes are arranged in another column at intervals, and one infrared light emitting diode and one red light emitting diode are arranged in still another column at intervals. The first direction is perpendicular to the second direction. The at least one photosensor includes two photosensors. The two photosensors are arranged at intervals in the second direction, and are respectively located on two sides of a smallest rectangular area in which the at least one light emitting component is located. In this design, a distance between a light emitting diode and a photosensor that are required in a detection process of the wearable device can be controlled based on a wearing state (for example, the wearable device is worn tightly or loosely) of the wearable device, that is, a reflection distance of light is controlled. For example, when the wearable device is worn loosely, the reflection distance of light is increased because an impact of ambient light is relatively large. When the wearable device is worn tightly, the reflection distance of light is reduced because an impact of ambient light is relatively small. This improves detection precision of the wearable device, and reduces power consumption.

According to a second aspect, the present invention provides a photoelectric pulse sensor component, where the photoelectric pulse sensor component includes a substrate; at least one light emitting component that is mounted on one side of the substrate and is configured to emit light on at least one optical wavelength band; at least one lens disposed on an out-light side of the at least one light emitting component, where each lens corresponds to at least one light emitting component, and each lens is capable of reducing a divergence angle of light emitted by the at least one light emitting component corresponding to the lens; at least one photosensor mounted on the substrate, where the at least one photosensor is mounted on a same side of the substrate as the at least one light emitting component, and is configured to receive the light emitted by the at least one light emitting component, to generate a sensing signal; and a control chip that is electrically connected to the at least one light emitting component and the at least one photosensor and is configured to: control each light emitting component in the at least one light emitting component to emit light, receive and process the sensing signal generated by the at least one photosensor, and output the processed sensing signal. In this design, a parameter such as an exercise heart rate, a resting heart rate, and oxygen saturation of a target creature (for example, a human body) may be detected based on the sensing signal that is output by the photoelectric pulse sensor component. In addition, the photoelectric pulse sensor component may be mounted in a wearable object, so that when the wearable object is worn on the target creature (for example, a human body), a function of detecting the parameter such as an exercise heart rate, a resting heart rate, and oxygen saturation of the target creature can be implemented. For example, the wearable object includes but is not limited to a watch, a wristband, shoes, glasses, a helmet, and the like.

### BRIEF DESCRIPTION OF DRAWINGS

FIG. 1 is a schematic structural diagram of a wearable device according to some embodiments of the present invention;
FIG. 2 is a schematic structural diagram of a device body of a wearable device in related technologies;
FIG. 3 is a schematic diagram of emitting light by a light emitting component according to some embodiments of the present invention;
FIG. 4a is a schematic diagram of emitting light when a wearable device has a lens according to some embodiments of the present invention;
FIG. 4b is another schematic diagram of emitting light when a wearable device has a lens according to some embodiments of the present invention;
FIG. 5 is a schematic diagram of optical contribution proportions of each layer of skin and a window glass when a wearable device has no lens and optical contribution proportions of each layer of skin and the window glass when the wearable device has a lens;
FIG. 6 is a schematic structural diagram of another wearable device according to some embodiments of the present invention;
FIG. 7 is a schematic structural diagram of a lens of the wearable device in FIG. 6;
FIG. 8a is a schematic structural diagram of still another wearable device according to some embodiments of the present invention;
FIG. 8b is a schematic structural diagram of still another wearable device according to some embodiments of the present invention;
FIG. 8c is a schematic diagram of emitting light when a Fresnel lens cooperates with a light emitting component according to some embodiments of the present invention;
FIG. 9 is a schematic structural diagram of still another wearable device according to some embodiments of the present invention;
FIG. 10 is a schematic structural diagram of still another wearable device according to some embodiments of the present invention;
FIG. 11 is a schematic structural diagram of still another wearable device according to some embodiments of the present invention;
FIG. 12 is a schematic structural diagram of still another wearable device according to some embodiments of the present invention;
FIG. 13 is a schematic structural diagram of still another wearable device according to some embodiments of the present invention; and
FIG. 14 is a schematic structural diagram of a photoelectric pulse sensor component according to some embodiments of the present invention.

### DESCRIPTION OF EMBODIMENTS

For ease of understanding, with reference to the accompanying drawings in this specification, the following describes in detail a wearable device and a photoelectric pulse sensor component that are provided in the embodiments of the present invention.

The embodiments of the present invention provide a wearable device and a photoelectric pulse sensor component. The wearable device may be directly worn on a human body, or integrated into clothes or an accessory of a person, and may detect a parameter such as an exercise heart rate, a resting heart rate, and oxygen saturation of the human body. The photoelectric pulse sensor component may be mounted in a wearable object, so that the wearable object has a function that is of detecting the parameter such as an exercise heart rate, a resting heart rate, and oxygen saturation and that is of the foregoing wearable device. For example, the wearable object includes but is not limited to a watch, a wristband, shoes, glasses, a helmet, and the like. This is not limited in the embodiments of the present invention.

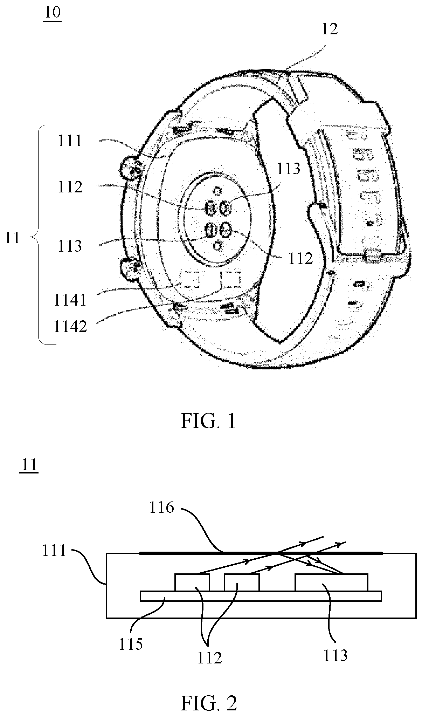

FIG. 1 is a schematic structural diagram of a wearable device according to an embodiment of the present invention. As shown in FIG. 1, in some embodiments of the present invention, a wearable device 10 may include a device body 11 and a flexible fastening strap 12 connected to two ends of the device body 11.

A shape and a material of the flexible fastening strap 12 are not limited in this embodiment of the present invention. For example, the flexible fastening strap 12 may be implemented by using one flexible strip, and two ends of the flexible strip are respectively connected to the two ends of the device body 11. Alternatively, the flexible fastening strap 12 may be implemented by using two flexible strips. One end of each flexible strip in the two flexible strips is connected to one end in the two ends of the device body 11, and the other end of each flexible strip in the two flexible strips is detachably connected by using a connection structure, for example, a clamping connection or a threaded connection.

When the wearable device 10 is worn on a human body (for example, a wrist), a ring is surrounded by the device body 11 and the flexible fastening strap 12, and the device body 11 may emit light on at least one optical wavelength band to an inner side of the ring. Herein, it should be noted that the ring in this embodiment is not an absolute standard circle. For example, the ring may be in a shape that matches a wrist shape and that approximates to a circle. In this way, the device body 11 may be in contact with the human body, so that when the device body 11 emits light on at least one optical wavelength band to the inner side of the ring, the light on the at least one optical wavelength band can enter the human body.

The device body 11 may further receive light reflected after the light on the at least one optical wavelength band enters the human body and is absorbed by blood and tissues of the human body, and may calculate at least one parameter such as an exercise heart rate, a resting heart rate, and oxygen saturation based on an intensity change of the reflected light based on photoelectric detection according to a principle that a blood volume changes with a pulse.

For example, as shown in FIG. 1, the device body 11 includes a housing 111, at least one light emitting component 112, at least one photosensor 113, a control chip 1141, and a processor 1142. The processor 1142 is connected to the control chip 1141. The control chip 1141 is connected to the at least one light emitting component 112 and the at least one photosensor 113. The at least one light emitting component 112, the at least one photosensor 113, the control chip 1141, and the processor 1142 are all mounted in the housing 111. A position that is on the housing and that corresponds to the at least one light emitting component 112 and the at least one photosensor 113 is transparent.

In a process of detecting a heart rate by using a PPG pulse wave method, the control chip 1141 controls the at least one light emitting component 112 to emit light on a specified optical wavelength band, light reflected after the light on the specified optical wavelength band passes through skin tissues is received by the at least one photosensor 113 and converted into a sensing signal. The control chip 1141 processes (for example, performs filtering processing and analog-to-digital conversion on) the sensing signal, and then outputs the processed sensing signal to the processor 1142. The processor 1142 may calculate a heart rate of a target creature (for example, a human body wearing the wearable device 10) based on the processed sensing signal. The heart rate includes an exercise heart rate and a resting heart rate.

It should be noted that, when the light on the specified optical wavelength band passes through the skin tissues and then is reflected to the photosensor 113, light intensity attenuates to a degree. When a part for measurement has no significant movement, absorption of light by muscles, bones, veins, other connective tissues, and the like basically does not change, but the blood is different. Because the blood flows in arteries, absorption of light by the arteries naturally changes. When the photosensor 113 converts light into a sensing signal, because the absorption of light by the arteries changes and the absorption of light by the other parts basically does not change, the obtained sensing signal includes a direct current signal and an alternating current signal. Finally, the control chip 1141 extracts the alternating current signal in the sensing signal, and outputs the alternating current signal to the processor 1142. The alternating current signal may reflect a blood flow characteristic. Therefore, the processor 1142 may calculate a heart rate. For example, the processor 1142 may obtain a quantity of peaks of the alternating current signal within specified time through analysis, to obtain a heart rate. For example, assuming that a quantity of peaks within consecutive 5 seconds is N, the heart rate is N × 12 bpm (beat per minute, beat per minute).

In some possible designs, the light on the specified optical wavelength band is green light, red light, or infrared light. The red light and the infrared light pass through the skin tissues more easily than the green light, and the green light entering the skin tissues is more easily absorbed by the blood. Therefore, considering various cases of skin, at least one of the green light, the red light, or the infrared light may be used through automatic switching. For example, when the skin has much melanin or sweat, the red light or the infrared light may be used, so that more light passes through the skin tissues. When the skin is relatively white or dry, the green light may be used to increase a change amplitude of the sensing signal to obtain a more precise measurement result.

In a process of detecting oxygen saturation by using a PPG pulse wave method, the control chip 1141 controls the at least one light emitting component 112 to emit red light and infrared light, light reflected after the red light and the infrared light pass through skin tissues is received by the at least one photosensor 113 and converted into a sensing signal. The control chip 1141 processes the sensing signal, and then outputs the processed sensing signal to the processor 1142. The processor 1142 may calculate oxygen saturation of a target creature (for example, a human body wearing the wearable device 10) based on the processed sensing signal. The oxygen saturation is a percentage of a volume of oxygenated hemoglobin bound to oxygen in the blood to a total volume of bindable hemoglobin, namely, a blood oxygen concentration in the blood, and is an important physiological parameter of respiration and circulation. When the red light and the infrared light are used for detection, the bindable hemoglobin more easily absorbs the red light and the oxygenated hemoglobin more easily absorbs the infrared light. Therefore, the bindable hemoglobin and the oxygenated hemoglobin may be respectively detected by using the red light and the infrared light. That is, the photosensor receives the reflected red light and converts the reflected red light into a first sensing signal, and receives the reflected infrared light to obtain a second sensing signal. Then, the control chip 1141 extracts an alternating current signal in the first sensing signal and an alternating current signal in the second sensing signal, and outputs the alternating current signals to the processor 1142. The processor 1142 calculates a corresponding ratio, namely, the oxygen saturation.

In some possible designs, the control chip 1141 may be integrated into the processor 1142, so that the control chip 1141 becomes a part of the processor 1142. In this way, a quantity of components can be reduced, to facilitate assembly of the wearable device, and simplify an internal structure of the wearable device.

Herein, it should be noted that, the processor 1142 may be a central processing unit (Central Processing Unit, CPU), or an application-specific integrated circuit (Application-Specific Integrated Circuit, ASIC), or may be configured as one or more integrated circuits implementing some embodiments of the present disclosure, for example, one or more DSPs or one or more field programmable gate arrays (Field Programmable Gate Array, FPGA).

In related technologies, as shown in FIG. 2, a substrate 115 is disposed in a housing 111, a light emitting component 112 and a photosensor 113 are disposed on the substrate 115, the light emitting component 112 and the photosensor 113 are located on a same side of the substrate 115, and the housing 111 has a window glass 116 through which a light ray passes. In the related technologies, light emitted by the light emitting component 112 has a relatively large divergence angle. When a large-angle light ray emitted by the light emitting component 112 is incident on a surface of the window glass 116, interface reflection is easily caused. As such, crosstalk light occurs on the surface of the window glass 116, and consequently light passing through the window glass 116 is reduced and light loss is relatively severe. In addition, most light is directly reflected by the window glass 116 to the photosensor 113. Consequently, an amount of light received by the photosensor 113 is saturated and detection cannot be normally performed. In addition, because the divergence angle of the emitted light is relatively large, a depth at which the light enters a human body after passing through the window glass 116 becomes shallow, less light is absorbed by blood, light utilization is reduced, and a proportion of desired light is reduced. This affects a detection result of a parameter such as a heart rate and oxygen saturation, reduces detection precision of the parameter such as a heart rate and oxygen saturation, and increases power consumption.

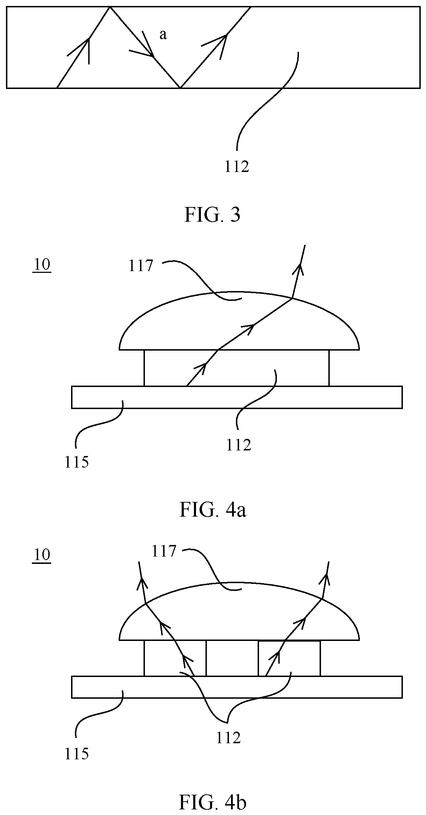

In addition, referring to FIG. 3, in the related technologies, a difference between a refractive index of the light emitting component 112 and a refractive index of air is relatively large. Consequently, a total reflection light ray a of light generated by the light emitting component 112 is generated inside the light emitting component 112, out-light efficiency is relatively low, and light utilization is further reduced.

To resolve the foregoing problem, some embodiments of the present invention provide a wearable device 10. Referring to FIG. 4a and FIG. 4b, the wearable device 10 includes a substrate 115, at least one light emitting component 112, and at least one lens 117.

The at least one light emitting component 112 is mounted on one side of the substrate 115, and is configured to emit light on at least one optical wavelength band. The at least one lens 117 is disposed on an out-light side of the at least one light emitting component 112, each lens 117 corresponds to at least one light emitting component 112, and each lens 117 is capable of reducing a divergence angle of light emitted by the at least one light emitting component 112 corresponding to the lens 117. For example, FIG. 4a shows an example in which each lens 117 corresponds to one light emitting component 112. FIG. 4b shows an example in which each lens 117 corresponds to two light emitting components 112. In this way, a light ray emitted by each light emitting component 112 can be concentrated in the center, so that a large-angle light ray emitted by the light emitting component 112 can be effectively used, a depth at which the light ray emitted by the light emitting component 112 enters a human body becomes deeper, and more light is absorbed by blood, that is, a proportion of effective light is increased. This can improve detection precision of a parameter such as a heart rate and oxygen saturation, and reduce power consumption.

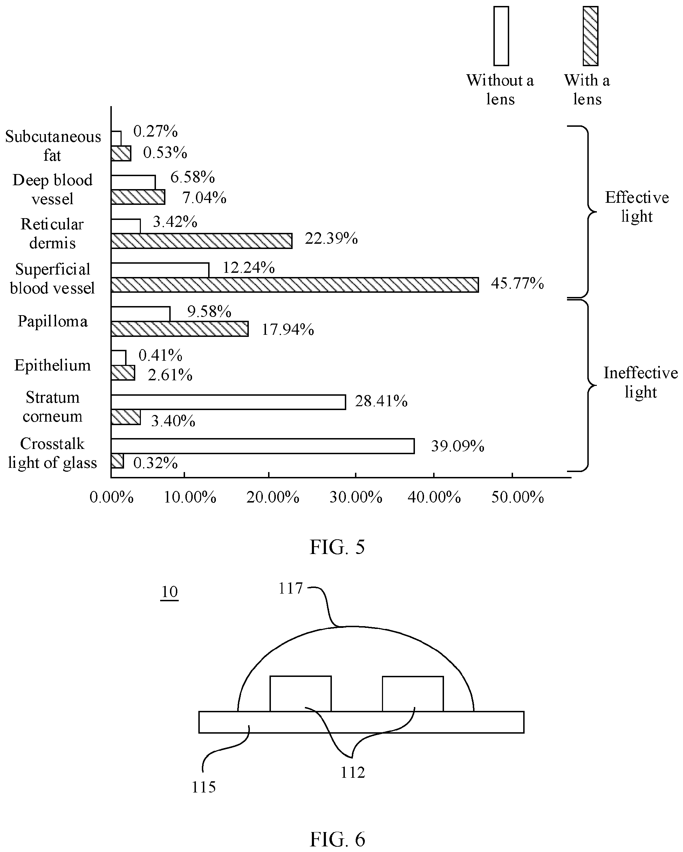

It should be noted that FIG. 5 shows optical contribution proportions of each layer of skin and a window glass when a wearable device has no lens and optical contribution proportions of each layer of skin and the window glass when the wearable device has a lens. As shown in FIG. 5, compared with a case in which the wearable device has no lens, when the wearable device has a lens, optical contribution proportions of crosstalk light of the window glass and stratum corneum of human skin are greatly decreased, but optical contribution proportions of superficial blood vessels and reticular dermis are greatly increased, and optical contribution proportions of deep blood vessels and subcutaneous fat are also increased. Therefore, when the wearable device has a lens, a proportion of effective light may be greatly increased, and a proportion of ineffective light may be reduced, so that a depth at which a light ray emitted by the light emitting component enters a human body becomes deeper, and more light is absorbed by blood. This can improve detection precision of a parameter such as a heart rate and oxygen saturation, and reduce power consumption.

It should be noted that, in a process of detecting the heart rate, the oxygen saturation, and the like, from "a light ray is emitted by the light emitting component 112" to "the light ray is received by the photosensor 113", a part is lost when the light ray passes through the window glass and each layer of skin. A proportion of the part, that is lost when the light ray passes through the window glass and each layer of skin, to a total amount of light emitted by the light emitting component 112 is referred to as an optical contribution proportion of the layer.

In a possible design, referring to FIG. 4a and FIG. 4b, each lens 117 is located on a side that is of one or more light emitting components 112 corresponding to the lens 117 and that is away from the substrate 115. In addition, each lens 117 is fastened to the one or more light emitting components 112 corresponding to the lens 117. For example, the lens 117 and the light emitting component 112 are relatively fastened in an adhesive manner. In this way, a structure of the lens 117 can be simple, and mounting and fastening can be convenient.

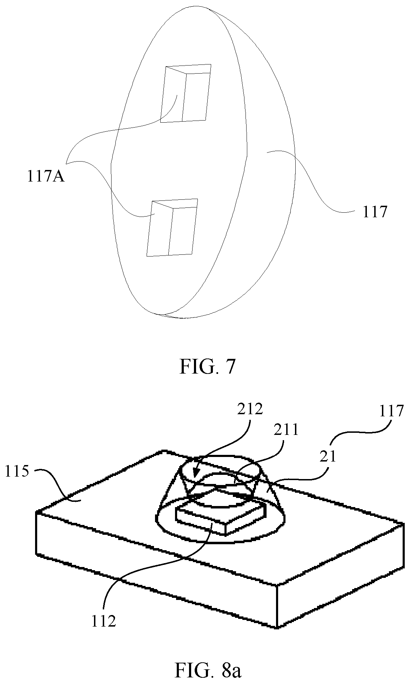

In another possible design, referring to FIG. 6 and FIG. 7, the at least one lens 117 is fastened to the substrate 115, a surface that is of each lens 117 and that is connected to the substrate 115 has at least one concave cavity 117A, at least one accommodation space is surrounded by the at least one concave cavity 117A and the substrate 115, and the at least one light emitting component 112 corresponding to each lens 117 is disposed in the at least one accommodation space. One or more light emitting components 112 may be accommodated in each accommodation space. For example, when a shape of each concave cavity 117A matches a shape of the light emitting component 112, each light emitting component 112 may be exactly embedded in a concave cavity, in other words, disposed in an accommodation space surrounded by the concave cavity 117A and the substrate 115. When the light emitting component 112 and the substrate 115 are relatively fastened, movement of a corresponding lens 117 in a direction parallel to the substrate may be limited by using the light emitting component 112. When the lens 117 and the substrate 115 are relatively fastened, movement of the light emitting component 112 may be limited. In addition, the light emitting component 112 can be further protected, so that the light emitting component 112 is not vulnerable to external moisture, and a service life of the light emitting component 112 is prolonged. In addition, the lens 117 is directly connected to the substrate 115, and the light emitting component 112 is located in a corresponding concave cavity 117A. Therefore, an overall thickness that exists in a direction perpendicular to the substrate 115 after the lens 117 and the substrate 115 are assembled can be further reduced.

In some possible designs, to further enhance a connection effect, glue may be further put between the substrate 115 and a surface, of the lens 117, facing the substrate 115, so that the lens 117 and the substrate 115 are relatively fastened. This can enhance firmness and reliability after the lens is connected to the substrate.

A shape of the concave cavity 117A is not limited in this embodiment of the present invention, provided that an accommodation space is surrounded by the concave cavity 117A and the substrate 115 to accommodate at least one light emitting component 112. For example, the concave cavity 117A is in a columnar shape or a conical frustum shape.

In some embodiments, referring to FIG. 6 and FIG. 7, the at least one lens 117 is a convex lens. For example, the lens 117 is a biconvex lens. Alternatively, the lens 117 is a plano-convex lens shown in FIG. 6 and FIG. 7. When the lens 117 is a plano-convex lens, a curved surface of the lens 117 is an out-light surface, to reduce a divergence angle of light emitted by the light emitting component 112, so that a light ray emitted by each light emitting component 112 is concentrated in the center. At least one concave cavity 117A may be disposed on a flat surface of the lens 117 (for example, FIG. 7 shows an example in which two concave cavities 117A are disposed on the flat surface of the lens 117). In addition, when the flat surface is connected to the substrate 115, an accommodation space for accommodating the light emitting component 112 may be formed between the concave cavity 117A and the substrate 115. In this way, the lens 117 can be conveniently mounted on the substrate 115, to improve reliability of a connection between the lens 117 and the substrate 115, and an overall thickness that exists in a direction perpendicular to the substrate after the lens 117 and the substrate 115 are assembled can be further reduced. In addition, the light emitting component 112 can be further protected, so that the light emitting component 112 is not vulnerable to external moisture, and a service life of the light emitting component 112 is prolonged.

In some embodiments, a refractive index of each lens is greater than a refractive index of air. This helps increase an angle of refraction of a light ray emitted by the lens, and further reduce a divergence angle of light emitted by the light emitting component, so that more emergent light rays are concentrated in the center. Therefore, a large-angle light ray emitted by the light emitting component is effectively used, so that a depth at which the light ray emitted by the light emitting component enters a human body becomes deeper, and more light is absorbed by blood, that is, a proportion of desired light is increased, to further improve detection precision of a parameter such as a heart rate and oxygen saturation, and reduce power consumption.

In some embodiments, referring to FIG. 6 and FIG. 7, each lens 117 is in contact with an out-light surface of the at least one light emitting component 112 corresponding to the lens 117. In this way, more emergent light rays can be extracted from the light emitting component 112, so that a total reflection light ray of light generated by the light emitting component 112 is not easily generated inside the light emitting component 112. This helps improve out-light efficiency of the light emitting component, reduce light loss, and improve light utilization.

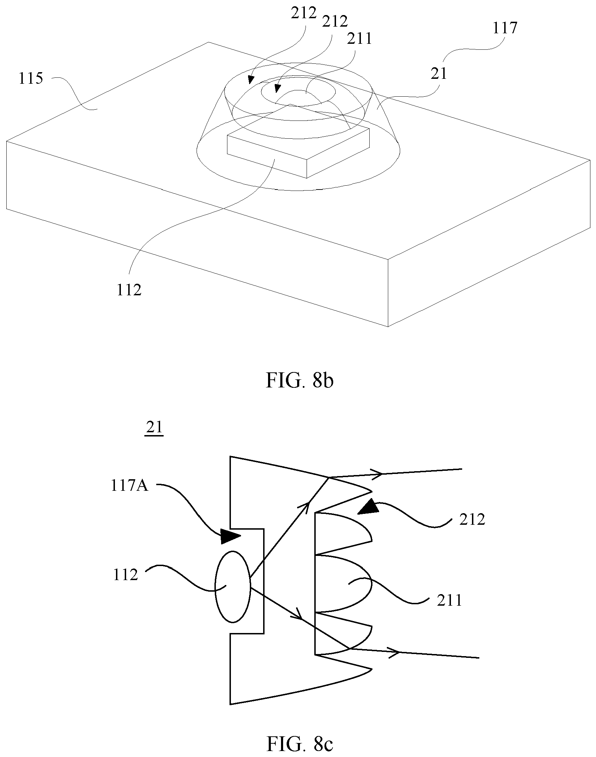

In some embodiments, referring to FIG. 8a to FIG. 8c, the at least one lens 117 is a Fresnel lens 21, and a protrusion 211 and at least one circle of groove 212 that has a sawtooth cross section and that is disposed around the protrusion 211 are provided on a surface that is of the Fresnel lens 21 and that faces away from at least one light emitting component 112 corresponding to the Fresnel lens 21. For example, one circle of groove 212 may be disposed, or two or more circles of grooves 212 may be disposed. This is not limited herein. A surface of a side, of the groove 212, close to the protrusion 211 is an out-light surface. As shown in FIG. 8c, the out-light surface may be a curved surface, so that the Fresnel lens 21 can reduce a divergence angle of light emitted by the light emitting component 112. In this design, a thickness of the lens 11 in a direction perpendicular to the substrate 115 can be reduced while the divergence angle of the light emitted by the light emitting component 112 is reduced, so that an internal structure of the wearable device is more compact, to miniaturize the wearable device.

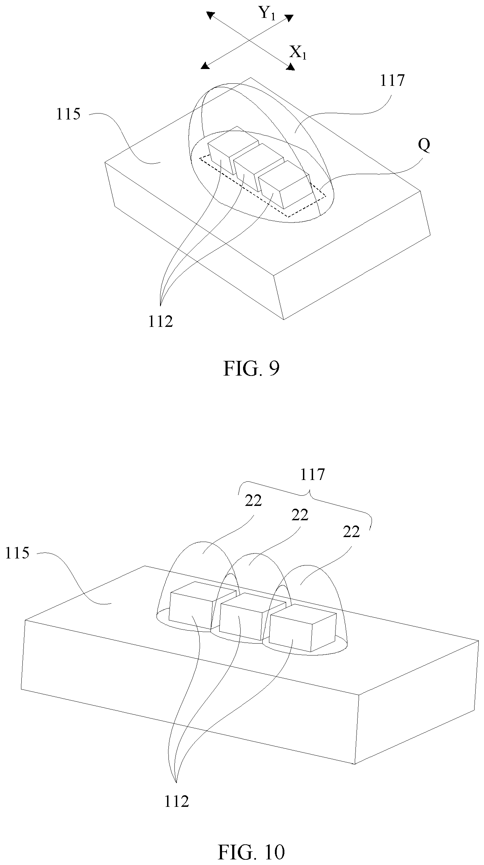

In some embodiments, referring to FIG. 9, a shape of the at least one lens 117 is a part of an ellipsoid, an orthographic projection of the part of the ellipsoid on the substrate 115 is an ellipse, and a major axis direction X₁ of the ellipse is parallel to a long side direction of a smallest rectangular area Q in which at least one light emitting component corresponding to the lens is located. The part of the ellipsoid may be a part that is of the ellipsoid and that is obtained through cutting off in a direction parallel to a major axis of the ellipsoid, or may be a part that is of the ellipsoid and that is obtained through cutting off in a direction parallel to a minor axis of the ellipsoid. It may be understood that the part of the ellipsoid may be a quarter of the ellipsoid, a semi-ellipsoid (a half of the ellipsoid), or three quarters of the ellipsoid. This is not limited herein.

For example, referring to FIG. 9, when the lens 117 is a semi-ellipsoid that is obtained through cutting off in the direction parallel to the major axis of the ellipsoid, because an orthographic projection of the semi-ellipsoid on the substrate 115 is an ellipse, a projection of the semi-ellipsoid on a plane perpendicular to the substrate 115 and parallel to the major axis direction X₁ is a first semi-ellipse, and a projection of the semi-ellipsoid on a plane perpendicular to the substrate 115 and perpendicular to the major axis direction X₁ is a second semi-ellipse. This helps reduce a divergence angle of light emitted by the light emitting component 112. In a possible design, a curvature of the second semi-ellipse is greater than a curvature of the first semi-ellipse. In this way, a size of the lens 117 in a minor axis direction Y₁ of the ellipse can be reduced. In addition, after light emitted by the light emitting component 112 passes through the lens 117, a divergence angle in the minor axis direction Y₁ is greater than a divergence angle in the major axis direction X₁. In this case, as shown in FIG. 9, the light emitting components 112 are sequentially arranged in the long side direction of the smallest rectangular area Q. This helps greatly reduce a divergence angle, of light emitted by the light emitting components 112, in the minor axis direction Y₁ at the same time, so that practicability is relatively high.

For example, when the lens 117 is a semi-ellipsoid that is obtained through cutting off in the direction parallel to the major axis of the ellipsoid, at least one concave cavity may be disposed on a cross section of the semi-ellipsoid, so that an accommodation space for accommodating the light emitting component 112 is formed when the lens 117 is connected to the substrate 115. In this way, the lens 117 can be conveniently mounted on the substrate 115, to improve reliability of a connection between the lens 117 and the substrate 115, and an overall thickness that exists in a direction perpendicular to the substrate after the lens 117 and the substrate 115 are assembled can be further reduced. In addition, the light emitting component 112 can be further protected, so that the light emitting component 112 is not vulnerable to external moisture, and a service life of the light emitting component 112 is prolonged.

In some embodiments, referring to FIG. 10, each lens 117 in the at least one lens 117 includes at least two sub-lenses 22, each sub-lens 22 is a part of a sphere or a part of an ellipsoid, an orthographic projection of the part of the sphere or the part of the ellipsoid on the substrate 115 is a circle, each sub-lens 22 corresponds to one light emitting component 112, and two adjacent sub-lenses 22 partially overlap. As shown in FIG. 10, three sub-lenses 22 are sequentially arranged, and every two adjacent sub-lenses 22 partially overlap. This helps reduce a size of the lens 117 in an arrangement direction of the at least two sub-lenses 22, to miniaturize the wearable device, and a divergence angle of light emitted by a corresponding light emitting component 112 can be reduced by using each sub-lens 22. Therefore, this helps a light ray emitted by each light emitting component 112 be concentrated in the center, so that a depth at which the light ray emitted by each light emitting component 112 enters a human body becomes deeper, more light is absorbed by blood, and a proportion of desired light of each light emitting component 112 is increased. This improves detection precision of a heart rate and oxygen saturation, and reduces power consumption.

The part of the sphere may be a part that is of the sphere and that is obtained through cutting off in a direction perpendicular to an axis direction of the sphere. The part of the ellipsoid may be a part that is of the ellipsoid and that is obtained through cutting off in a direction perpendicular to a major axis of the ellipsoid. It may be understood that the part of the sphere may be a quarter of the sphere, a hemisphere (a half of the sphere), or three-quarters of the sphere, and the part of the ellipsoid may be a quarter of the ellipsoid, a semi-ellipsoid (a half of the ellipsoid), or three-quarters of the ellipsoid. This is not limited herein.

For example, referring to FIG. 10, when the sub-lens 22 is a semi-ellipsoid that is obtained through cutting off in the direction perpendicular to the major axis of the ellipsoid, because an orthographic projection of the semi-ellipsoid on the substrate 115 is a circle, a projection of the semi-ellipsoid on any plane perpendicular to the substrate 115 is a semi-ellipse. This helps further reduce a divergence angle of light emitted by the light emitting component 112.

For example, referring to FIG. 10, when the sub-lens 22 is a semi-ellipsoid that is obtained through cutting off in the direction perpendicular to the major axis of the ellipsoid, at least one concave cavity may be disposed on a cross section of the semi-ellipsoid, so that an accommodation space for accommodating the light emitting component 112 is formed when the sub-lens 22 is connected to the substrate 115. In this way, the sub-lens 22 can be conveniently mounted on the substrate 115, to improve reliability of a connection between the sub-lens 22 and the substrate 115, and an overall thickness that exists in a direction perpendicular to the substrate after each sub-lens 22 and the substrate 115 are assembled can be further reduced. In addition, a corresponding light emitting component 112 can be further protected by using each sub-lens 22, so that each light emitting component 112 is not vulnerable to external moisture, and a service life of each light emitting component 112 is prolonged.

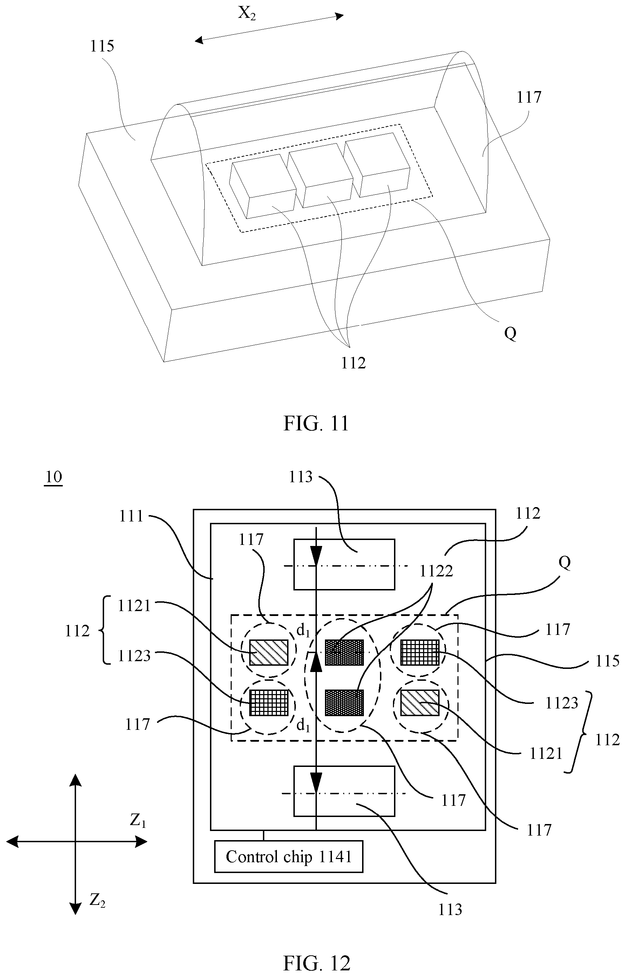

In some embodiments, referring to FIG. 11, a shape of the at least one lens 117 is a part of a cylinder, the part of the cylinder is a part that is obtained through cutting off in a direction parallel to an axis direction of the cylinder, and a cross section of the part of the cylinder in a direction perpendicular to the substrate and perpendicular to the axis direction X₂ of the cylinder is a part of a circle or a part of an ellipse. As shown in FIG. 11, an orthographic projection of the part of the cylinder on the substrate is a rectangle, and a long side direction of the rectangle is parallel to a long side direction of a smallest rectangular area Q in which at least one light emitting component corresponding to the lens is located.

The part obtained through cutting off may be a half, one third, or a quarter of the cylinder. This is not specifically limited herein. The cross section of the lens 117 in the direction perpendicular to the substrate and perpendicular to the axis direction X₂ of the cylinder is a part of a circle or a part of an ellipse. Therefore, a divergence angle, of light emitted by each light emitting component 112, in the direction perpendicular to the axis direction X₂ of the cylinder can be effectively reduced, and a size of the lens 117 in the axis direction X₂ of the cylinder can be reduced.

For example, at least one concave cavity may be disposed on a flat surface, of the lens 117, parallel to the axis direction X₂ of the cylinder, so that an accommodation space for accommodating the light emitting component 112 is formed when the lens 117 is connected to the substrate 115. In this way, the lens 117 can be conveniently mounted on the substrate 115, to improve reliability of a connection between the lens 117 and the substrate 115, and an overall thickness that exists in a direction perpendicular to the substrate after the lens 117 and the substrate 115 are assembled can be further reduced. In addition, the light emitting component 112 can be further protected, so that the light emitting component 112 is not vulnerable to external moisture, and a service life of the light emitting component 112 is prolonged.

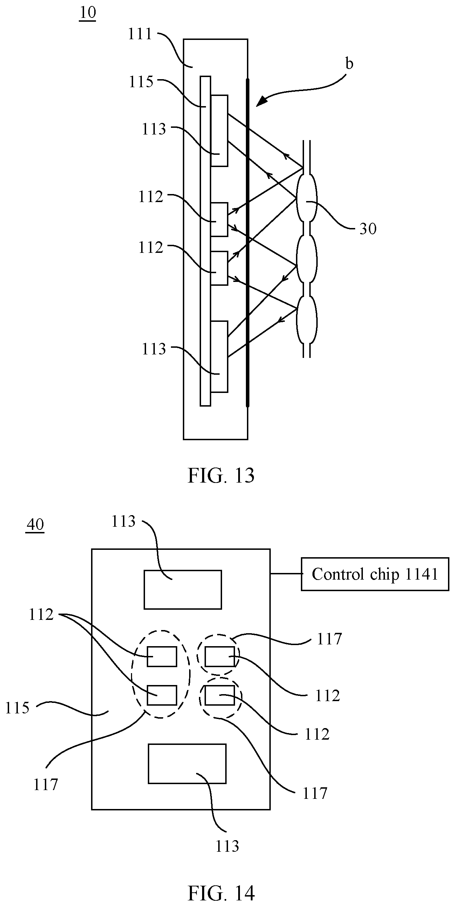

In some embodiments, referring to FIG. 12 and FIG. 13, the at least one light emitting component 112 includes at least one red light emitting diode 1121, at least one green light emitting diode 1122, and at least one infrared light emitting diode 1123. The wearable device 10 further includes: at least one photosensor 113 mounted on the substrate 115, where the at least one photosensor 113 is mounted on a same side of the substrate 115 as the at least one light emitting component 112, and is configured to receive the light emitted by the at least one light emitting component 112, to generate a sensing signal; a control chip 1141 that is electrically connected to the at least one light emitting component 112 and the at least one photosensor 113 and is configured to: control a specified light emitting diode (for example, the at least one red light emitting diode 1121, the at least one green light emitting diode 1122, and/or the at least one infrared light emitting diode 1123) in the at least one light emitting component 112 to emit light, receive and process the sensing signal generated by the at least one photosensor 113, and output the processed sensing signal; and a housing 111, where the substrate 115 and the control chip 1141 are mounted in the housing 111, and a position b that is on the housing 111 and that corresponds to the at least one light emitting component 112 and the at least one photosensor 113 is transparent. For example, the position b on the housing 111 may be a through opening, or may be a protective cover made of a transparent material such as glass. When the position b on the housing 111 is a protective cover, moisture can be prevented from entering the housing, so that components in the wearable device are not vulnerable to the moisture.

Referring to FIG. 13, components (for example, the substrate 115, the light emitting component 112, the lens 117, and the photosensor 113) other than the housing 111 of the wearable device 10 are integrated into the housing 111. This has advantages of a simple structure and ease of portability. In addition, light emitted by the light emitting component (for example, a human body) may be incident into a target creature (for example, a human body) by using the transparent position on the housing, and light reflected by blood 30 of the human body is received by the photosensor, so that a plurality of parameters such as an exercise heart rate, a resting heart rate, oxygen saturation, heart rate variability, sleep, and a blood pressure of the target creature (for example, a human body) can be detected. For example, the resting heart rate, the heart rate variability, the sleep, and the blood pressure may be detected by using the red light emitting diode 1121, the green light emitting diode 1122, or the infrared light emitting diode 1123; the oxygen saturation may be detected by emitting light by the red light emitting diode 1121 and the infrared light emitting diode 1123 simultaneously; and the exercise heart rate may be detected by using the green light emitting diode 1122.

On this basis, in a possible design, as shown in FIG. 12, the at least one light emitting component 112 includes two red light emitting diodes 1121, two green light emitting diodes 1122, and two infrared light emitting diodes 1123. In a first direction Z₁, one red light emitting diode 1121, one green light emitting diode 1122, and one infrared light emitting diode 1123 are sequentially arranged in a row at intervals, and the other red light emitting diode 1121, the other green light emitting diode 1122, and the other infrared light emitting diode 1123 are sequentially arranged in another row at intervals. In a second direction Z₂, one red light emitting diode 1121 and one infrared light emitting diode 1123 are arranged in a column at intervals, two green light emitting diodes 1122 are arranged in another column at intervals, and one infrared light emitting diode 1123 and one red light emitting diode 1121 are arranged in still another column at intervals. The first direction Z₁ is perpendicular to the second direction Z₂. The at least one photosensor 113 includes two photosensors 113. The two photosensors 113 are arranged at intervals in the second direction Z₂, and are respectively located on two sides of a smallest rectangular area Q in which the at least one light emitting component is located.

In this design, the wearable device may detect a plurality of parameters such as an exercise heart rate, a resting heart rate, and oxygen saturation of a target creature (for example, a human body). In addition, after different photosensors 113 each convert received light into a sensing signal, the wearable device may further determine a wearing state of the wearable device based on the sensing signal generated by each photosensor, for example, the wearable device is worn tightly or loosely.

It should be noted that, when the wearable device is worn tightly, the transparent position b on the housing 111 can be fully in contact with a surface of the human body. In this case, interference of ambient light can be reduced. When the wearable device is worn loosely, the transparent position b on the housing 111 may be in less contact with a surface of the human body. In this case, interference of ambient light may be increased. On this basis, distances between each light emitting diode and the photosensors are different. Therefore, when the wearable device is worn loosely, a light emitting diode and a photosensor that are relatively far away from each other may be selected, to increase a reflection distance of light. This can reduce interference of ambient light. In addition, when the wearable device is worn tightly, a light emitting diode and a light sensor that are relatively close to each other may be selected, to reduce a reflection distance of light, so that a depth at which a light ray emitted by the light emitting diode enters the human body becomes deeper. This improves detection precision of the wearable device, and reduces power consumption. As shown in FIG. 12, a distance between a green light emitting diode 1122 and one photosensor 113 is d₁, and a distance between the green light emitting diode 1122 and the other photosensor 113 is d₂. For example, d₁ may be 28 mm, and d₂ may be 40 mm.

On this basis, for example, referring to FIG. 12, the at least one lens in the wearable device 10 includes one first-type lens 117 and four second-type lenses 117. A shape of the first-type lens 117 is a part of an ellipsoid, an orthographic projection of the part of the ellipsoid on the substrate is an ellipse, and a major axis direction of the ellipse is parallel to an arrangement direction of two green light emitting diodes 1122. Both the two green light emitting diodes 1122 are located between the first-type lens 117 and the substrate 115. Each second-type lens 117 is a part of an ellipsoid, and an orthographic projection of the part of the ellipsoid on the substrate 115 is a circle. Four light emitting diodes other than the two green light emitting diodes 1122 are separately located between the second-type lens 117 and the substrate 115. In this design, a divergence angle of light emitted by each light emitting component can be effectively reduced by using the first-type lens 117 and the second-type lens 117, so that a light ray emitted by each light emitting component 112 is concentrated in the center. Therefore, a large-angle light ray emitted by the light emitting component 112 is effectively used, so that a depth at which the light ray emitted by the light emitting component 112 enters a human body becomes deeper, and more light is absorbed by blood, that is, a proportion of effective light is increased. This effectively improves detection precision of a heart rate and oxygen saturation, and reduces power consumption.

Referring to FIG. 14, some embodiments of the present invention further provide a photoelectric pulse sensor component 40. The photoelectric pulse sensor component 40 includes a substrate 115, at least one light emitting component 112 mounted on one side of the substrate 115, at least one lens 117 disposed on an out-light side of the at least one light emitting component 112, at least one photosensor 113 mounted on the substrate 115, and a control chip 1141.

The at least one light emitting component 112 is configured to emit light on at least one optical wavelength band.

Each lens 117 corresponds to at least one light emitting component 112, and each lens 117 is capable of reducing a divergence angle of light emitted by the at least one light emitting component 112 corresponding to the lens 117.

The at least one photosensor 113 is mounted on a same side of the substrate 115 as the at least one light emitting component 112, and is configured to receive the light emitted by the at least one light emitting component 112, to generate a sensing signal.

The control chip 1141 is electrically connected to the at least one light emitting component 112 and the at least one photosensor 113, and is configured to: control each light emitting component 112 in the at least one light emitting component 112 to emit light, receive and process the sensing signal generated by the at least one photosensor 113, and output the processed sensing signal, so that a component such as a processor can calculate at least one parameter such as an exercise heart rate, a resting heart rate, and oxygen saturation of a target creature based on the sensing signal.

In this design, the parameter such as an exercise heart rate, a resting heart rate, and oxygen saturation of the target creature (for example, a human body) may be detected by using the photoelectric pulse sensor component 40. In addition, the photoelectric pulse sensor component 40 may be mounted in a wearable object, so that when the wearable object is worn on the target creature (for example, a human body), a function of detecting the parameter such as an exercise heart rate, a resting heart rate, and oxygen saturation of the target creature can be implemented. For example, the wearable object includes but is not limited to a watch, a wristband, shoes, glasses, a helmet, and the like. This is not limited in the embodiments of the present invention.

In the descriptions of the implementations, the described specific features, structures, materials, or characteristics may be combined in a proper manner in any one or more of the embodiments or examples.

The foregoing descriptions are merely specific implementations of the present invention, but are not intended to limit the protection scope of the present invention. Any variation or replacement within the technical scope disclosed in the present invention shall fall within the protection scope of the present invention. Therefore, the protection scope of the present invention shall be subject to the protection scope of the claims.

## Claims

1. A wearable device, comprising a substrate (115), and further comprising:
at least one light emitting component (112) that is mounted on one side of the substrate and is configured to emit light on at least one optical wavelength band; and
at least one lens (117) disposed on an out-light side of the at least one light emitting component, wherein each lens corresponds to at least one light emitting component, and each lens is capable of reducing a divergence angle of light emitted by the at least one light emitting component corresponding to the lens,
wherein each lens in the at least one lens comprises at least two sub-lenses (22), each sub-lens is a part of a sphere or a part of an ellipsoid, **characterized in that** an orthographic projection of the part of the sphere or the part of the ellipsoid on the substrate is a circle; and
each sub-lens corresponds to one light emitting component, and two adjacent sub-lenses partially overlap.

2. The wearable device according to claim 1, wherein the at least one lens is fastened to the substrate, a surface that is of each lens and that is connected to the substrate has at least one concave cavity, and at least one accommodation space is surrounded by the at least one concave cavity and the substrate; and
the at least one light emitting component corresponding to each lens is disposed in the at least one accommodation space.

3. The wearable device according to claim 1, wherein a refractive index of each lens is greater than a refractive index of air.

4. The wearable device according to claim 1, wherein each lens is in contact with an out-light surface of the at least one light emitting component corresponding to the lens.

5. The wearable device according to claim 1, wherein the at least one light emitting component (112) comprises at least one red light emitting diode, at least one green light emitting diode, and at least one infrared light emitting diode; and
the wearable device further comprises:
at least one photosensor (113) mounted on the substrate, wherein the at least one photosensor is mounted on a same side of the substrate as the at least one light emitting component, and is configured to receive the light emitted by the at least one light emitting component, to generate a sensing signal;
a control chip (1141) that is electrically connected to the at least one light emitting component and the at least one photosensor and is configured to: control a specified light emitting diode in the at least one light emitting component to emit light, receive and process the sensing signal generated by the at least one photosensor, and output the processed sensing signal; and
a housing (111), wherein the substrate and the control chip are mounted in the housing, and a position that is on the housing and that corresponds to the at least one light emitting component and the at least one photosensor is transparent.

6. The wearable device according to claim 5, wherein the at least one light emitting component comprises two red light emitting diodes, two green light emitting diodes, and two infrared light emitting diodes;
in a first direction, one red light emitting diode, one green light emitting diode, and one infrared light emitting diode are sequentially arranged in a row at intervals, and the other red light emitting diode, the other green light emitting diode, and the other infrared light emitting diode are sequentially arranged in another row at intervals;
in a second direction, one red light emitting diode and one infrared light emitting diode are arranged in a column at intervals, two green light emitting diodes are arranged in another column at intervals, and one infrared light emitting diode and one red light emitting diode are arranged in still another column at intervals, wherein the first direction is perpendicular to the second direction; and
the at least one photosensor comprises two photosensors, wherein the two photosensors are arranged at intervals in the second direction, and are respectively located on two sides of a smallest rectangular area in which the at least one light emitting component is located.

7. A wearable device according to claim 1, further comprising:
at least one photosensor (113) mounted on the substrate, wherein the at least one photosensor is mounted on a same side of the substrate as the at least one light emitting component, and is configured to receive the light emitted by the at least one light emitting component, to generate a sensing signal; and
a control chip (1141) that is electrically connected to the at least one light emitting component and the at least one photosensor and is configured to: control each light emitting component in the at least one light emitting component to emit light, receive and process the sensing signal generated by the at least one photosensor, and output the processed sensing signal.

## Patentansprüche

1. Tragbare Vorrichtung, umfassend ein Substrat (115) und ferner umfassend:
mindestens eine Licht abstrahlende Komponente (112), die auf einer Seite des Substrats montiert ist und dafür ausgelegt ist, Licht in mindestens einem optischen Wellenlängenband abzustrahlen; und
mindestens eine Linse (117), die auf einer Licht abstrahlenden Seite der mindestens einen Licht abstrahlenden Komponente angeordnet ist, wobei jede Linse mindestens einer Licht abstrahlenden Komponente entspricht und jede Linse in der Lage ist, einen Divergenzwinkel des von der mindestens einen Licht abstrahlenden Komponente, die der Linse entspricht, abgestrahlten Lichts zu verkleinern,
wobei jede Linse in der mindestens einen Linse mindestens zwei Unterlinsen (22) umfasst, wobei jede Unterlinse ein Teil einer Kugel oder ein Teil eines Ellipsoids ist,
**dadurch gekennzeichnet, dass**
eine orthografische Projektion des Teils der Kugel oder des Teils des Ellipsoids auf das Substrat ein Kreis ist; und
jede Unterlinse einer Licht abstrahlenden Komponente entspricht und zwei benachbarte Unterlinsen sich teilweise überlappen.

2. Tragbare Vorrichtung gemäß Anspruch 1, wobei die mindestens eine Linse an dem Substrat befestigt ist, eine Oberfläche, die zu einer jeweiligen Linse gehört und die mit dem Substrat verbunden ist, mindestens einen konkaven Hohlraum aufweist und mindestens ein Aufnahmeraum von dem mindestens einen konkaven Hohlraum und dem Substrat umgeben ist; und
die mindestens eine jeder Linse entsprechende Licht abstrahlende Komponente in dem mindestens einen Aufnahmeraum angeordnet ist.

3. Tragbare Vorrichtung gemäß Anspruch 1, wobei ein Brechungsindex jeder Linse größer ist als ein Brechungsindex von Luft.

4. Tragbare Vorrichtung gemäß Anspruch 1, wobei jede Linse mit einer Licht abstrahlenden Oberfläche der mindestens einen der Linse entsprechenden Licht abstrahlenden Komponente in Kontakt ist.

5. Tragbare Vorrichtung gemäß Anspruch 1, wobei die mindestens eine Licht abstrahlende Komponente (112) mindestens eine rote Leuchtdiode, mindestens eine grüne Leuchtdiode und mindestens eine Infrarot-Leuchtdiode umfasst; und
die tragbare Vorrichtung ferner umfasst:
mindestens einen Fotosensor (113), der auf dem Substrat montiert ist, wobei der mindestens eine Fotosensor auf derselben Seite des Substrats wie die mindestens eine Licht abstrahlende Komponente montiert ist und dafür ausgelegt ist, das von der mindestens einen Licht abstrahlenden Komponente abgestrahlte Licht zu empfangen, um ein Sensorsignal zu erzeugen;
einen Steuerchip (1141), der elektrisch mit der mindestens einen Licht abstrahlenden Komponente und dem mindestens einen Fotosensor verbunden ist und dafür ausgelegt ist: eine bestimmte Leuchtdiode in der mindestens einen Licht abstrahlenden Komponente so zu steuern, dass sie Licht abstrahlt, das von dem mindestens einen Fotosensor erzeugte Sensorsignal zu empfangen und zu verarbeiten und das verarbeitete Sensorsignal auszugeben; und
ein Gehäuse (111), wobei das Substrat und der Steuerchip in dem Gehäuse und an einer Position, die sich auf dem Gehäuse befindet und die der mindestens einen Licht abstrahlenden Komponente entspricht, montiert sind und der mindestens eine Fotosensor transparent ist.

6. Tragbare Vorrichtung gemäß Anspruch 5, wobei die mindestens eine Licht abstrahlende Komponente zwei rote Leuchtdioden, zwei grüne Leuchtdioden und zwei Infrarot-Leuchtdioden umfasst;
in einer ersten Richtung eine rote Leuchtdiode, eine grüne Leuchtdiode und eine Infrarot-Leuchtdiode hintereinander in einer Reihe in Abständen angeordnet sind und die andere rote Leuchtdiode, die andere grüne Leuchtdiode und die andere Infrarot-Leuchtdiode hintereinander in einer anderen Reihe in Abständen angeordnet sind;
in einer zweiten Richtung eine rote Leuchtdiode und eine Infrarot-Leuchtdiode in einer Spalte in Abständen angeordnet sind, zwei grüne Leuchtdioden in einer anderen Spalte in Abständen angeordnet sind und eine Infrarot-Leuchtdiode und eine rote Leuchtdiode in noch einer weiteren Spalte in Abständen angeordnet sind, wobei die erste Richtung senkrecht zur zweiten Richtung verläuft; und
der mindestens eine Fotosensor zwei Fotosensoren umfasst, wobei die beiden Fotosensoren in Abständen in der zweiten Richtung angeordnet sind und sich jeweils auf zwei Seiten eines kleinsten rechteckigen Bereichs befinden, in dem sich die mindestens eine Licht abstrahlende Komponente befindet.

7. Tragbare Vorrichtung gemäß Anspruch 1, ferner umfassend:
mindestens einen Fotosensor (113), der auf dem Substrat montiert ist, wobei der mindestens eine Fotosensor auf derselben Seite des Substrats wie die mindestens eine Licht abstrahlende Komponente montiert ist und dafür ausgelegt ist, das von der mindestens einen Licht abstrahlenden Komponente abgestrahlte Licht zu empfangen, um ein Sensorsignal zu erzeugen; und
einen Steuerchip (1141), der elektrisch mit der mindestens einen Licht abstrahlenden Komponente und dem mindestens einen Fotosensor verbunden ist und dafür ausgelegt ist: jede Licht abstrahlende Komponente der mindestens einen Licht abstrahlenden Komponente so zu steuern, dass sie Licht abstrahlt, das von dem mindestens einen Fotosensor erzeugte Sensorsignal zu empfangen und zu verarbeiten und das verarbeitete Sensorsignal auszugeben.

## Revendications

1. Dispositif pouvant être porté, comprenant un substrat (115), et comprenant en outre :
au moins un composant émetteur de lumière (112) qui est monté sur un côté du substrat et est configuré pour émettre de la lumière sur au moins une bande de longueurs d'onde optiques ; et
au moins une lentille (117) disposée sur un côté hors de la lumière de l'au moins un composant émetteur de lumière, où chaque lentille correspond à au moins un composant émetteur de lumière, et chaque lentille est apte à réduire un angle de divergence de la lumière émise par l'au moins un composant émetteur de lumière correspondant à la lentille,
où chaque lentille dans l'au moins une lentille comprend au moins deux sous-lentilles (22), chaque sous-lentille fait partie d'une sphère ou fait partie d'un ellipsoïde, **caractérisé en ce que**
une projection orthographique de la partie de la sphère ou de la partie de l'ellipsoïde sur le substrat est un cercle ; et
chaque sous-lentille correspond à un composant émetteur de lumière, et deux sous-lentilles adjacentes se chevauchent partiellement.

2. Dispositif pouvant être porté selon la revendication 1, où l'au moins une lentille est fixée au substrat, une surface qui appartient à chaque lentille et qui est raccordée au substrat a au moins une cavité concave, et au moins un espace de logement est entouré de l'au moins une cavité concave et du substrat ; et
l'au moins un composant émetteur de lumière correspondant à chaque lentille est disposé dans l'au moins un espace de logement.

3. Dispositif pouvant être porté selon la revendication 1, où un indice de réfraction de chaque lentille est plus grand qu'un indice de réfraction de l'air.

4. Dispositif pouvant être porté selon la revendication 1, où chaque lentille est en contact avec une surface hors de la lumière de l'au moins un composant émetteur de lumière correspondant à la lentille.

5. Dispositif pouvant être porté selon la revendication 1, où l'au moins un composant émetteur de lumière (112) comprend au moins une diode électroluminescente rouge, au moins une diode électroluminescente verte, et au moins une diode électroluminescente à infrarouges ; et
le dispositif pouvant être porté comprend en outre :
au moins un photocapteur (113) monté sur le substrat, où l'au moins un photocapteur est monté sur un même côté du substrat que l'au moins un composant émetteur de lumière, et est configuré pour recevoir la lumière émise par l'au moins un composant émetteur de lumière, afin de générer un signal de captage ;
une puce de commande (1141) qui est connectée électriquement à l'au moins un composant émetteur de lumière et à l'au moins un photocapteur, et est configurée pour : commander une diode électroluminescente spécifiée dans l'au moins un composant émetteur de lumière pour émettre de la lumière, recevoir et traiter le signal de captage généré par l'au moins un photocapteur, et produire le signal de captage traité ; et
un logement (1111), où le substrat et la puce de commande sont monté/es dans le logement, et une position qui se situe sur le logement et qui correspond à l'au moins un composant émetteur de lumière et l'au moins un photocapteur est transparent.

6. Dispositif pouvant être porté selon la revendication 5, où l'au moins un composant émetteur de lumière comprend deux diodes électroluminescentes rouges, deux diodes électroluminescentes vertes, et deux diodes électroluminescentes à infrarouges ;
dans une première direction, une diode électroluminescente rouge, une diode électroluminescente verte et une diode électroluminescente à infrarouges sont agencées séquentiellement dans une rangée à certains intervalles, et l'autre diode électroluminescente rouge, l'autre diode électroluminescente verte et l'autre diode électroluminescente à infrarouges sont agencées séquentiellement dans une autre rangée à certains intervalles ;
dans une deuxième direction, une diode électroluminescente rouge et une diode électroluminescente à infrarouges sont agencées dans une colonne à certains intervalles, deux diodes électroluminescentes vertes sont agencées dans une autre colonne à certains intervalles, et une diode électroluminescente à infrarouges et une diode électroluminescente rouge sont agencées dans encore une autre colonne à certains intervalles, la première direction étant perpendiculaire à la deuxième direction ; et
l'au moins un photocapteur comprend deux photocapteurs, les deux photocapteurs étant agencés à certains intervalles dans la deuxième direction, et étant localisés respectivement sur deux côtés d'une zone rectangulaire la plus petite dans laquelle l'au moins un composant émetteur de lumière est localisé.

7. Dispositif pouvant être porté selon la revendication 1, comprenant en outre :
au moins un photocapteur (113) monté sur le substrat, où l'au moins un photocapteur est monté sur un même côté du substrat que l'au moins un composant émetteur de lumière, et est configuré pour recevoir la lumière émise par l'au moins un composant émetteur de lumière, afin de générer un signal de captage ; et
une puce de commande (1141) qui est connectée électriquement à l'au moins un composant émetteur de lumière et à l'au moins un photocapteur, et est configurée pour : commander chaque composant émetteur de lumière dans l'au moins un composant émetteur de lumière pour émettre de la lumière, recevoir et traiter le signal de captage généré par l'au moins un photocapteur, et produire le signal de captage traité.
